# EUROPÄISCHE PATENTSCHRIFT

(11) **EP 1 990 021 B1**
(45) Veröffentlichungstag und Bekanntmachung des Hinweises auf die Patenterteilung: **06.07.2011**
(21) Anmeldenummer: 07009316.6
(22) Anmeldetag: 09.05.2007
(51) Int. Cl.: A61B 19/00

(54) **Medizinisches Instrument mit separater Sendereinheit zur Ansteuerung einer behandlungsunterstützenden Software, sowie Instrumentensystem**
Medical instrument with separate transmission unit for controlling software to support treatment and instrument system
Instrument médical doté d'une unité émettrice séparée destinée à la commande d'un logiciel de gestion, tout comme système d'instruments

(43) Veröffentlichungstag der Anmeldung: 12.11.2008
(73) Patentinhaber: BrainLAB AG, 85622 Feldkirchen (DE)
(72) Erfinder: Hager, Stefan, 80687 München (DE); Urban, Alexander, 85570 Markt Schwaben (DE)
(74) Vertreter: Schwabe - Sandmair - Marx

(56) Entgegenhaltungen:
- EP-A- 1 302 172

## Beschreibung

Die vorliegende Erfindung betrifft ein medizinisches Instrument mit einer Steuerungsvorrichtung zur Ansteuerung einer behandlungsunterstützenden Software. Ferner betrifft sie ein System solcher Instrumente.

In der computerunterstützten Chirurgie werden dem Behandelnden - meist von einem Navigations- bzw. Trackingsystem im Operationssaal - unterschiedliche Bildausgaben zur Verfügung gestellt. Diese Bildausgaben zeigen Patientendatensätze oder zumindest Teile davon, also z.B. dreidimensionale oder Schnittwiedergaben von Patientenkörperteilen. Die Patientendatensätze können entweder durch bildgebende Verfahren wie z.B. CT- oder MR-Tomographie, Röntgen, Ultraschall oder Fluoroskopie, oder durch bildlose Verfahren, wie das Abgreifen einer Knochenoberfläche mittels eines registrierten Zeigerwerkzeugs oder mit Laserabtastung erzeugt werden. Ferner können im Rahmen der Bildunterstützung beispielsweise Instrumente oder Behandlungseinrichtungen im Positionsverhältnis zu den Patientendaten gezeigt werden, um so dem Behandelnden eine visuelle Unterstützung zu geben.

Je nach Behandlungsfortschritt wird es oft notwendig, einen speziellen Teil der Softwareunterstützung auf der Bildschirmausgabe anzuzeigen, nämlich den Teil mit den Funktionen, die für den momentanen Behandlungsschritt gerade erforderlich sind. Man könnte auch sagen, dass die Software aus verschiedenen "Seiten" besteht, die im Laufe der Behandlung durchwechseln. Oft ist es notwendig, während der Behandlung bestimmte Software-Seiten anzuwählen, und dies wird oft mittels eines Eingabegerätes wie einer Maus oder einer Tastatur oder mittels eines berührungsempfindlichen Bildschirms bewerkstelligt. Auch Fussschalter, virtuelle Tastaturen oder Sprachsteuerungen sind bekannt.

Oftmals wird es jedoch von den Behandelnden als sehr störend empfunden, dass sie sich neben ihrer Operationstätigkeit noch zusätzlich dem Umschalten der Software widmen müssen, insbesondere wenn sie dazu ein separates Gerät zur Dateneingabe zu betätigen haben. Darunter leidet die Akzeptanz der ansonsten als hoch vorteilhaft angesehenen Bildunterstützung.

Um für Registrierungspointer einen Signalgeber direkt am Instrument bereitzustellen, so dass bei der Berührung der Patienten-"Oberfläche" ein Signal abgegeben wird, sind Instrumente mit berührungsempfindlichen Spitzen vorgestellt worden, beispielsweise in der EP 1 302 172 A1. Der Einsatz solcher Instrumente beschränkt sich allerdings auf Registrierungszwecke.

Die WO 01/54558 A2 offenbart ein Instrument gemäß dem Oberbegriff von Anspruch 1, bei dem ein Zeigegerät (Pointer) verwendet wird, das Software-Steuerungsknöpfe aufweist. Der hier gezeigte Pointer sendet aktiv über LEDs Navigations-Trackingimpulse aus, so dass er geortet und positionell verfolgt werden kann. Die Steuerungsvorrichtung für die Software ist fest mit dem Pointer integriert, was die Sterilisierung erschwert. Die Spitzen des Instruments sind auswechselbar, was zwar unterschiedliche Einsatzmöglichkeiten gestattet, jedoch immer eine neue Kalibrierung des Instruments erfordert.

Es ist die Aufgabe der vorliegenden Erfindung, ein medizinisches Instrument mit einer Steuerungsvorrichtung zur Ansteuerung einer behandlungsunterstützenden Software bereitzustellen, welches die oben genannten Nachteile des Standes der Technik überwindet. Insbesondere soll mindestens eines der vorliegenden Ziele erreicht werden: breite Einsatzmöglichkeit des Instruments; einfache Sterilisierbarkeit; einfache Handhabbarkeit; Möglichkeit zur Bereitstellung kalibrierter Instrumente.

Diese Aufgabe wird durch ein medizinisches Instrument gemäß dem Anspruch 1 gelöst. Die Unteransprüche definieren vorteilhafte Ausführungsformen der Erfindung.

Ein medizinisches Instrument gemäß der Erfindung weist die Merkmale von Anspruch 1 auf.

Mit anderen Worten weist das medizinische Instrument eine auswechselbare bzw. vom Instrument getrennt bereitstellbare Sendereinheit auf, die im Instrument, d.h. in dessen Instrumentenkörper untergebracht werden kann. Eine solche separate, einsetzbare und entnehmbare Sendereinheit bietet viele Vorteile, und einer davon betrifft die Tatsache, dass die Sendereinheit zum Sterilisieren des Instruments aus diesem entnommen werden kann, so dass keine Probleme mit schwer sterilisierbaren Senderteilen oder Energieversorgungen mehr auftreten. Das Instrument wird durch die Bereitstellung der inneren Aufnahme für die Sendereinheit in seiner äußeren Form nicht verändert und kann wie gewohnt verwendet werden, natürlich mit dem zusätzlichen Vorteil, dass es zur Ansteuerung der Software benutzt werden kann. Eine einzige Sendereinheit kann für mehrere Instrumente verwendet werden, die bereits vorkalibriert sind, d.h. für welche die Position des funktionalen Teils (z.B. Instrumentenspitze) schon vorab im Navigationssystem hinterlegt und bekannt ist.

Bei einer Ausführungsform des Instruments weist der Schnellverschluss, einen Klappverschluss, eine Kappenverschluss oder eine Schieberverschluss auf. Solche Schnellverschlüsse garantieren ein einfaches Handling und eine schnelle Entnehmbarkeit bzw. Einsetzbarkeit für die Sendereinheit.

Der Verschluss für die Innenaufnahme kann abgedichtet sein, und bei einer bevorzugten Ausführungsform ist die Sendereinheit eine drahtlose Sendereinheit. Besonders im letzteren Fall ist es von Vorteil, wenn eine elektrische Energieversorgung für die Sendereinheit mit der Sendereinheit einsetzbar und entnehmbar ist, insbesondere zusammen mit der Sendereinheit vorgesehen oder an ihr angeordnet ist. Das Instrument kann dann unabhängig und kabellos betrieben werden, und die schwer zu sterilisierende Sendereinheit befindet sich im Inneren des Instruments und stellt deshalb kein Sterilitätsproblem mehr dar.

Gemäß einer weiteren Ausführungsvariante ist die Betätigungseinrichtung an der Sendeeinheit angeordnet und mit ihr einsetzbar und entnehmbar. Sie kann mindestens einen Bestätigungsschalter aufweisen und/oder eine Auswahlvorrichtung für unterschiedliche Software-Merkmale, die mit dem Bestätigungsschalter integriert oder separat von ihm vorgesehen ist. Ein solcher Bestätigungsschalter würde beispielsweise der "Enter"-Taste einer Tastatur oder einer Maus entsprechen. Beispiele für Betätigungseinrichtungen, die mit dem erfindungsgemäßen Instrument benutzt werden können, sind ein Druckknopf, ein Scrollrad, speziell mit Druckschalter, ein Joystick, speziell mit Druckschalter oder ein Kippschalter, wobei diese Liste natürlich nicht abschließend ist und die Erfindung auch andere Betätigungs- oder Bestätigungseinrichtungen benutzen kann und umfasst.

Bei einer Ausgestaltung des erfindungsgemäßen Instruments sind an dem Gehäuse des Instruments, insbesondere auch im Verschluss für die Innenaufnahme, Durchgänge für Betätigungseinrichtungen vorgesehen, die an der Sendereinheit angeordnet sind und nach außen aus dem Instrumentengehäuse hervorstehen. Dabei sind über den Durchgängen am Instrumentengehäuse Abdeckungen, insbesondere flexible Schutzabdeckungen, angeordnet, welche die Betätigungseinrichtungen aufnehmen, um die Abgeschlossenheit der Innenaufnahme aufrecht zu erhalten.

Gemäß einer weiteren Ausführungsform ist das medizinische Instrument so aufgebaut, dass die Betätigungseinrichtung bzw. die Betätigungseinrichtungen einen ersten Teil aufweisen, der am Instrumentengehäuse, insbesondere auch am Verschluss für die Innenaufnahme, angeordnet ist und das Gehäuse bzw. den Verschluss nach außen dicht abschließt, sowie einen zweiten Teil, der an der Sendereinheit angeordnet ist und auf die Betätigung des ersten Teils reagiert. Der erste Teil schließt das Instrument also steril nach außen ab und kann trotzdem als Betätigungseinrichtung dienen, während der zweite Teil an der Sendereinheit auf die Betätigung des ersten Teils reagiert, also beispielsweise durch einen Druck mit diesem verbunden wird oder ansonsten bei der Betätigung des ersten Teils mitbetätigt wird.

Das medizinische Instrument ist gemäß einer Ausführungsform ein mit Reflektorenmarkern versehenes Instrument, das mit einem passiven, medizintechnischen Navigations- und Trackingsystem geortet und verfolgt werden kann.

Gemäß einem weiteren Aspekt betrifft die vorliegende Erfindung ein Instrumentensystem mit mehreren Instrumenten, wie sie oben in verschiedenen Ausführungsformen beschrieben worden sind. Diese Instrumente weisen Innenaufnahmen auf, welche die gleiche Sendereinheit aufnehmen können, und dem System ist mindestens eine separate Sendereinheit zugeordnet. Hier kommt der schon oben angesprochene Vorteil zur Geltung, dass dieselbe Sendereinheit in verschiedenen, vorkalibrierten Instrumenten verwendet werden kann, und der Behandelnde erhält damit die Freiheit, gerade das Instrument zur Software-Steuerung auszuwählen, welches er im jeweiligen Operationsstadium bzw. die meiste Zeit über benutzt.

Bei einer vorteilhaften Ausführungsform ist die Erfindung ein Zeigerinstrument bzw. ein Pointer, der durch ein Kamerasystem (Trackingsystem) unter Verwendung von Referenzmarkern getrackt werden kann, die an dem Pointer angebracht sind. Dadurch wird es möglich, die Position der Pointerspitze in Datensätzen des Patienten zu detektieren. Unter Verwendung der Betätigungseinrichtung im Pointergriff kann die Software, welche die Patientendaten verarbeitet, betätigt werden, so dass sie spezielle Schritte ausführt, die bisher manuell angewählt werden mussten (z.B. durch einen Touch-Screen-Monitor des Navigationssystems). Die Übertragung der notwendigen Daten erfolgt vorteilhafterweise kabellos, und die Vorrichtung wird durch eine Energieversorgung betrieben, die im Pointer angeordnet ist, so dass keine Kabel an dem System vorgesehen werden müssen. Ein solcher Pointer, der Informationen über den gewünschten Software-Schritt übertragen kann, könnte verschiedene Pointereinrichtungen miteinander kombinieren und so die Verwendung der Software vereinfachen, beispielsweise bei Schritten wie dem Setzen einer Trajektorie oder dem Akquirieren von Punkten, und zwar indem einfach ein Knopf am Pointer betätigt wird. Das Navigationssystem bzw. das System, auf dem die behandlungsunterstützende Software läuft, hat einen empfangsbereiten Empfänger, der die Signale der Sendereinheit (Transmitter) empfangen kann. In einer Ausführungsform ist das System deshalb ein System, das nur in einer Richtung arbeitet, also ein One-Way-Kommunikationssystem, das auf einer kabellosen Kommunikation basiert. Die Sendereinheit ist entnehmbar, so dass die Basiseinheit (medizinisches Instrument, wie z.B. ein Pointer) separat sterilisierbar ist.

Die Erfindung wird im Weiteren anhand einer Ausführungsform näher erläutert. Sie kann alle hierin beschriebenen Merkmale einzeln sowie in jedweder sinnvollen Kombination umfassen. In den beiliegenden Zeichnungen zeigen:
- Figur 1: ein erfindungsgemäßes medizinisches Instrument mit aufgeklappter Innenaufnahme und entnommener Sendereinheit;
- Figuren 2 und 3 Figur 4: verschiedene Stadien beim Einsetzen der Sendereinheit; und das erfindungsgemäße Instrument mit integrierter bzw. eingebrachter Sendereinheit.

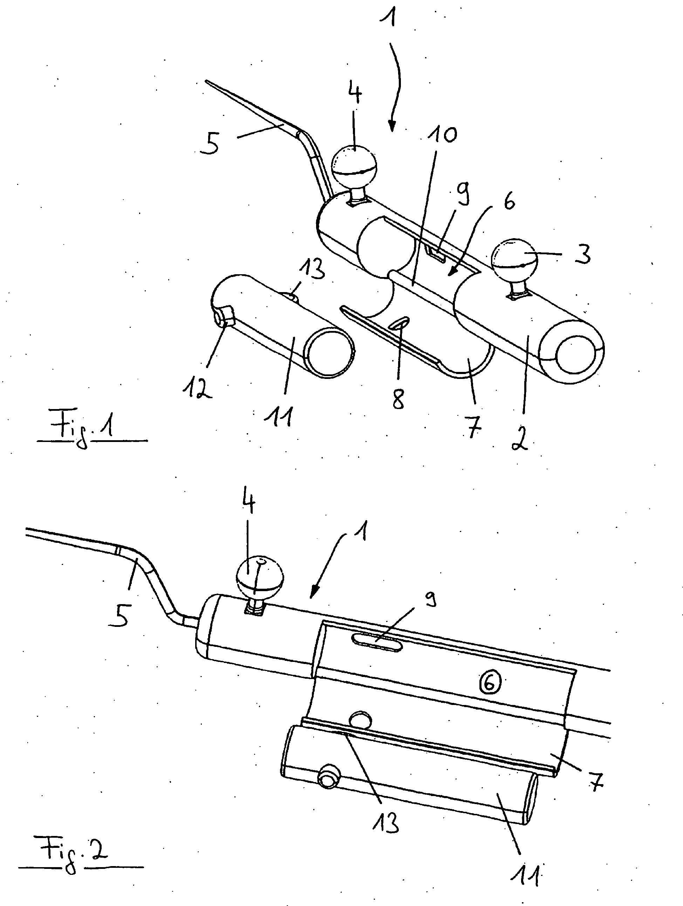

In der Figur 1 sind die Einzelteile des erfindungsgemäßen Instruments, hier eines Pointers 1 im Einzelnen bezeichnet. Der Pointer 1 hat einen Griff 2, der eine Art Gehäuse bildet. Am Griff 2 sind Navigations-Referenzmarker 3 und 4 angeordnet, hier als Reflektorenmarker bzw. Kugel-Reflektoren ausgebildet. Die Zeigerspitze des Instruments zeigt das Bezugszeichen 5 an.

Etwa in seinem Mittelbereich weist das Instrument bzw. der Griff 2 eine Innenaufnahme bzw. Ausnehmung 6 auf, hier eine zylinderförmigen Hohlraum. Zum Abschluss dieses Hohlraums dient die am Scharnier 10 schwenkbare Klappe 7. Die Klappe 7 und die Aufnahme 6 weisen Durchgangsöffnungen 8 und 9 auf, auf die später noch eingegangen wird.

Mit dem Instrument ist in Figur 1 auch die Sendereinheit 11 dargestellt, die ebenfalls zylinderförmig ausgebildet ist und in die Innenaufnahme 6 passt, wobei die Betätigungseinrichtungen, nämlich ein Druckknopf 12 und ein Scrollrad 13, in den Durchgangslöchern 8 und 9 zu liegen kommen können. Sie stehen damit aus dem Gehäuse, welches durch den Griff 2 gebildet wird, etwas hervor. Die Sendereinheit 11, in welcher eine Energieversorgung (z.B. Akkumulator, Batterie oder Piezo-System) integriert ist, ist eine drahtlose Sendereinheit zur Übertragung von Kommunikationssignalen. Sie sendet Funksignale an ein hier nicht dargestelltes Navigationssystem, welches - beispielsweise über einen Navigationsbildschirm - eine ansteuerbare Unterstützungssoftware bereitstellt. Vorteilhafterweise wird dasselbe Navigationssystem dazu benutzt, das Instrument 1 mit Hilfe eines medizintechnischen Trackingsystems über die Reflektorenmarker 3 und 4 zu orten und positionell zu verfolgen.

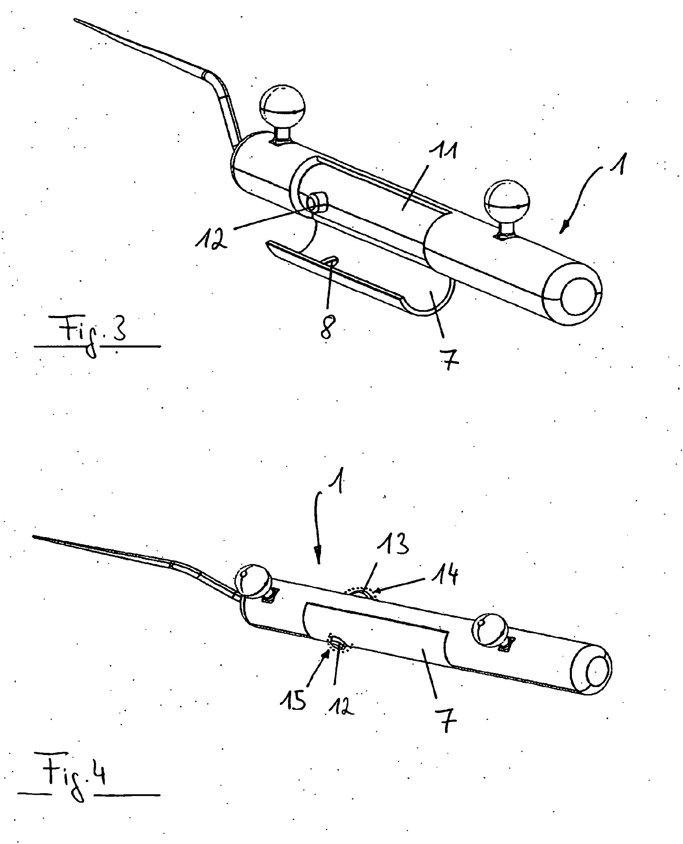

Die Figuren 2 und 3 zeigen, wie die Sendereinheit 11 in die Innenaufnahme 6 des Instruments 1 eingesetzt werden kann, wobei die Sendereinheit 11 zunächst so ausgerichtet wird, dass das Scrollrad 13 zur Durchgangsöffnung 9 hin ausgerichtet ist (Figur 2), worauf dann das Einsetzen erfolgen kann, so dass der Zustand der Figur 3 erreicht wird, in welchem die Sendereinheit 11 in der Innenaufnahme 6 liegt. Der Druckknopf 12 (Bestätigungs- oder Enter-Knopf) liegt dann so ausgerichtet, dass er beim Schließen der Klappe 7 durch die Durchgangsöffnung 8 hindurch tritt.

In der Figur 4 ist das mit der Sendereinheit ausgestattete chirurgische Instrument 1 zu sehen, wobei die Klappe 7 geschlossen ist und die Betätigungseinrichtungen, nämlich der Druckknopf 8 und das Scrollrad 13 durch ihre jeweiligen Durchgangslöcher etwas von der Oberfläche des Instruments hervorstehen, so dass sie betätigt werden können. Über dem Knopf 8 und dem Scroll-Rad 13 sind in der Figur 4 unterbrochene Linien 14, 15 dargestellt, welche den sterilen äußeren Gehäuseabschluss darstellen sollen. Die Vorrichtungen 14, 15 können flexible Schutzabdeckungen sein, die für den Fall bereitgestellt werden, wo beispielsweise der Knopf 12 fest an der Sendereinheit 11 angebracht ist. Durch die Abdeckung 15 bleibt das Äußere des Instrumentengehäuses steril. Die Abdeckungen können flexibel genug sein, so dass beispielsweise das Scroll-Rad 13 noch in eine Richtung (z.B. gegen eine Federspannung) gedrückt werden kann, um einen Scroll-Vorgang auszulösen. Symbolisch können die strichpunktierten Abdeckungen aber auch Betätigungseinrichtungen anzeigen, die einen dichten Gehäuseabschluss garantieren oder beispielsweise nur einen ersten Teil der Betätigungseinrichtung bilden, während der zweite Teil ein abgreifender Sensor an der Sendereinheit 11 ist, der die am Gehäuse befindlichen Betätigungseinrichtungen in Eingriff nimmt oder auf deren Druck reagiert.

Das Instrument kann nun verwendet werden, beispielsweise als RegistrierungsPointer. Dazu wird mittels des Scroll-Rades 13 die Registrierungs-Seite der Software ausgewählt, dann wird mit der Spitze des Instruments ein Registrierungspunkt am Patienten angefahren und mit dem Druckknopf 8 wird bestätigt, dass die angefahrene Position erreicht worden ist. Ein Registrierungspunkt kann damit in der Software gesetzt werden. Ein anderes Beispiel wäre die Auswahl einer Seite für das Setzen einer Behandlungs-Trajektorie mit dem Scrollrad 13, das Ausrichten des Pointers in Richtung der Trajektorie und die Bestätigung der Richtung mittels des Druckknopfes 8.

Wenn eine Anzahl von Instrumenten mit Innenaufnahmen ausgestattet werden, die einander in ihren Abmessungen entsprechen, kann die Sendereinheit 11 - wie schon dargestellt - zusammen mit mehreren unterschiedlichen, für die Navigation vorkalibrierten Instrumenten verwendet werden, beispielsweise auch mit einem Skalpell.

## Patentansprüche

1. Medizinisches Instrument (1) mit einer Steuerungsvorrichtung zur Ansteuerung einer behandlungsunterstützenden Software, wobei die Steuerungsvorrichtung einen Sender (11) zum Senden von Software-Ansteuerungssignalen und mindestens einer Betätigungseinrichtung (12, 13) zur Betätigung des Senders (11) aufweist, wobei der Sender als separate Sendereinheit (11) vorgesehen ist, **dadurch gekennzeichnet, dass** das Instrument (1) eine mit einem Schnellverschluss (7) verschließbare Innenaufnahme (6) aufweist, wobei die Sendereinheit (11) in die Innenaufnahme (6) einsetzbar und aus ihr entnehmbar ist.

2. Medizinisches Instrument nach Anspruch 1, **dadurch gekennzeichnet, dass** der Schnellverschluss (7) zum Verschließen der Innenaufnahme (6) einen Klappverschluss, einen Kappenverschluss oder einen Schieberverschluss aufweist.

3. Medizinisches Instrument nach Anspruch 1 oder 2, **dadurch gekennzeichnet, dass** der Verschluss (7) für die Innenaufnahme (6) abgedichtet ist.

4. Medizinisches Instrument nach einem der Ansprüche 1 bis 3, **dadurch gekennzeichnet, dass** die Sendereinheit (11) eine drahtlose Sendereinheit ist.

5. Medizinisches Instrument nach einem der Ansprüche 1 bis 4, **dadurch gekennzeichnet, dass** eine elektrische Energieversorgung für die Sendereinheit (11) mit der Sendereinheit (11) einsetzbar und entnehmbar ist, insbesondere zusammen mit der Sendereinheit (11) vorgesehen oder an ihr angeordnet ist.

6. Medizinisches Instrument nach einem der Ansprüche 1 bis 5, **dadurch gekennzeichnet, dass** die Betätigungseinrichtung (12, 13) an der Sendereinheit (11) angeordnet und mit ihr einsetzbar und entnehmbar ist.

7. Medizinisches Instrument nach einem der Ansprüche 1 bis 6, **dadurch gekennzeichnet, dass** die Betätigungseinrichtung mindestens einen Bestätigungsschalter (12) aufweist.

8. Medizinisches Instrument nach einem der Ansprüche 1 bis 7, **dadurch** gekenntzeichnet, das die Betätigungseinrichtung eine Auswahlvorrichtung für unterschiedliche Software-Merkmale aufweiset, die mit dem Bestätigungsschalter integriert oder separat von ihm vorgesehen ist.

9. Medizinisches Instrument nach einem der Ansprüche 1 bis 8, **dadurch gekennzeichnet, dass** die Betätigungseinrichtung eine oder mehrere der folgenden Vorrichtungen umfasst:
- einen Druckknopf (12);
- ein Scrollrad (13), speziell mit Druckschalter;
- einen Joystick, speziell mit Druckschalter;
- einen. Kippschalter.

10. Medizinisches Instrument nach einem der Ansprüche 1 bis 9, **dadurch gekennzeichnet, dass** an dem Gehäuse des Instruments, insbesondere auch im Verschluss (7) für die Innenaufnahme (6) Durchgänge für Betätigungseinrichtungen (12, 13) vorgesehen sind, die an der Sendereinheit (11) vorgesehen sind und nach außen aus dem Instrumentengehäuse hervorstehen, wobei über den Durchgängen am Instrumentengehäuse Abdeckungen angeordnet sind, welche die Betätigungseinrichtungen aufnehmen, insbesondere flexible Schutzabdeckungen.

11. Medizinisches Instrument nach einem der Ansprüche 1 bis 9, **dadurch gekennzeichnet, dass** die Betätigungseinrichtung bzw. die Betätigungseinrichtungen einen ersten Teil aufweisen, der am Instrumentengehäuse, insbesondere auch am Verschluss (7) für die Innenaufnahme (6), angeordnet ist und das Gehäuse bzw. den Verschluss nach außen dicht abschließt, sowie einen zweiten Teil, der an der Sendereinheit (11) angeordnet ist und auf die Betätigung des ersten Teils reagiert.

12. Medizinisches Instrument nach einem der Ansprüche 1 bis 11, **dadurch gekennzeichnet, dass** es ein mit Reflektorenmarkern (3, 4) versehenes Instrument (1) ist, das mit einem passiven, medizintechnischen Navigations- und Trackingsystem geortet und verfolgt werden kann.

13. Instrumentensystem mit mehreren Instrumenten gemäß einem der vorhergehenden Ansprüche, die Innenaufnahmen (6) aufweisen, welche die gleiche Sendereinheit (11) aufnehmen können, und mit mindestens einer separaten Sendereinheit (11).

## Claims

1. A medical instrument (1) comprising a control device for controlling a treatment-assisting software, wherein the control device comprises a transmitter (11) for transmitting software control signals and at least one activating means (12, 13) for activating the transmitter (11), wherein the transmitter is provided as a separate transmitter unit (11), **characterised in that** the instrument (1) comprises an interior receptacle (6) which can be closed using a quick-release shutter (7), wherein the transmitter unit (11) can be inserted into and removed from the interior receptacle (6).

2. The medical instrument according to claim 1, **characterised in that** the quick-release shutter (7) comprises a hinged shutter, a cap shutter or a sliding shutter, for closing the interior receptacle (6).

3. The medical instrument according to claim 1 or 2, **characterised in that** the shutter (7) for the interior receptacle (6) is sealed.

4. The medical instrument according to any one of claims 1 to 3, **characterised in that** the transmitter unit (11) is a wireless transmitter unit.

5. The medical instrument according to any one of claims 1 to 4, **characterised in that** an electrical energy supply for the transmitter unit (11) can be inserted and removed with the transmitter unit (11) and is in particular provided together with the transmitter unit (11) or arranged on it.

6. The medical instrument according to any one of claims 1 to 5, **characterised in that** the activating means (12, 13) is arranged on the transmitter unit (11) and can be inserted and removed with it.

7. The medical instrument according to any one of claims 1 to 6, **characterised in that** the activating means comprises at least one confirming switch (12).

8. The medical instrument according to any one of claims 1 to 7, **characterised in that** the activating means comprises a selecting device for different software features which is integrated with the confirming switch or is provided separately from it.

9. The medical instrument according to any one of claims 1 to 8, **characterised in that** the activating means comprises one or more of the following devices:
- a push button (12);
- a scroll wheel (13), specifically with a push switch;
- a joystick, specifically with a push switch;
- a rocker switch.

10. The medical instrument according to any one of claims 1 to 9, **characterised in that** apertures are provided on the casing of the instrument, in particular also in the shutter (7) for the interior receptacle (6), for activating means (12, 13) which are provided on the transmitter unit (11) and protrude outwards from the instrument casing, wherein coverings, in particular flexible protective coverings, are arranged over the apertures on the instrument casing and accommodate the activating means.

11. The medical instrument according to any one of claims 1 to 9, **characterised in that** the activating means comprises and/or comprise a first part which is arranged on the instrument casing, in particular also on the shutter (7) for the interior receptacle (6), and seals the casing and/or the shutter tight against the outside, and a second part which is arranged on the transmitter unit (11) and responds to the activation of the first part.

12. The medical instrument according to any one of claims 1 to 11, **characterised in that** it is an instrument (1) which is provided with reflector markers (3, 4) and can be localised and tracked using a passive medical navigation and tracking system.

13. An instrument system, comprising: a plurality of instruments in accordance with any one of the preceding claims, which comprise interior receptacles (6) which can accommodate the same transmitter unit (11); and at least one separate transmitter unit (11).

## Revendications

1. Instrument médical (1) comportant un dispositif de commande pour commander un logiciel de traitement, le dispositif de commande comportant un émetteur (11) pour envoyer des signaux de commande de logiciel et au moins un dispositif d'activation (12, 13) pour activer l'émetteur (11), l'émetteur étant réalisé sous la forme d'une unité émettrice séparée (11), **caractérisé en ce que** l'instrument (1) comporte un logement intérieur (6) pouvant être fermé par un élément de fermeture rapide (7), l'unité émettrice (11) pouvant être placée dans le logement intérieur (6) et en être retirée.

2. Instrument médical selon la revendication 1, **caractérisé en ce que** l'élément de fermeture rapide (5) comporte un couvercle à charnière, un capuchon ou un couvercle coulissant pour fermer le logement intérieur (6).

3. Instrument médical selon la revendication 1 ou 2, **caractérisé en ce que** l'élément de fermeture (7) du logement intérieur (6) est rendu étanche.

4. Instrument médical selon l'une quelconque des revendications 1 à 3, **caractérisé en ce que** l'unité émettrice (11) est une unité émettrice sans fil.

5. Instrument médical selon l'une quelconque des revendications 1 à 4, **caractérisé en ce qu'**une alimentation en énergie électrique pour l'unité émettrice (11) peut être insérée et retirée avec l'unité émettrice (11), en particulier est prévue pour être associée à l'unité émettrice (11) ou est agencée sur elle.

6. Instrument médical selon l'une quelconque des revendications 1 à 5, **caractérisé en ce que** le dispositif d'activation (12, 13) est agencé sur l'unité émettrice (11) et peut être inséré et retiré avec elle.

7. Instrument médical selon l'une quelconque des revendications 1 à 6, **caractérisé en ce que** le dispositif d'activation comporte au moins un interrupteur d'activation (12).

8. Instrument médical selon l'une quelconque des revendications 1 à 7, **caractérisé en ce que** le dispositif d'activation comporte un dispositif de sélection pour différentes caractéristiques de logiciel, lequel dispositif de sélection est intégré dans l'interrupteur d'activation ou est prévu séparément de celui-ci.

9. Instrument médical selon l'une quelconque des revendications 1 à 8, **caractérisé en ce que** le dispositif d'activation comporte un ou plusieurs des dispositifs suivants :
- un bouton-poussoir (12),
- une molette de défilement (13), spécialement avec un interrupteur à poussoir,
- un manche à balai, spécialement avec un interrupteur à poussoir,
- un interrupteur à bascule.

10. Instrument médical selon l'une quelconque des revendications 1 à 9, **caractérisé en ce que** sur le boîtier de l'instrument, en particulier également dans l'élément de fermeture (7) pour le logement intérieur (6), sont prévus des passages pour des dispositifs d'activation (12, 13), lesquels passages sont prévus sur l'unité émettrice (11) et font saillie vers l'extérieur à partir du boîtier d'instrument, dans lequel des couvercles, en particulier des couvercles de protection souples, sont agencés sur le boîtier d'instrument à travers les passages, lesquels couvercles reçoivent les dispositifs d'activation.

11. Instrument médical selon l'une quelconque des revendications 1 à 9, **caractérisé en ce que** le dispositif d'activation ou les dispositifs d'activation comportent une première partie qui est agencée sur le boîtier d'instrument, en particulier également sur l'élément de fermeture (7) pour le logement intérieur (6), et ferme le boîtier ou l'élément de fermeture de manière étanche par rapport à l'extérieur, ainsi qu'une seconde partie qui est agencée sur l'unité émettrice (11) et qui réagit en cas d'activation de la première partie.

12. Instrument médical selon l'une quelconque des revendications 1 à 11, **caractérisé en ce qu'**il s'agit d'un instrument (1) muni de marqueurs de réflexion (3, 4), lequel instrument peut être localisé et suivi à l'aide d'un système de navigation et de repérage médico-technique, passif.

13. Système d'instruments comportant plusieurs instruments selon l'une quelconque des revendications précédentes, lesquels instruments comportent des logements intérieurs (6) qui peuvent recevoir la même unité émettrice (11), et au moins une unité émettrice (11) séparée.
